# EUROPEAN PATENT APPLICATION

(11) **EP 1 982 730 A1**
(43) Date of publication of application: **22.10.2008**
(21) Application number: 08154751.5
(22) Date of filing: 17.04.2008
(51) Int. Cl.: A61K 45/06, A61P 35/00, A61P 35/02, A61K 31/205

(54) **Pharmaceutical composition comprising l-carnitine and an antagonist of the membrane adenosine receptors, derivatives and/or pharmaceutically acceptable salts thereof, and their use in the preparation of drugs with non-toxic effect to inhibit the proliferation of neoplastic cells**

(30) Priority: 19.04.2007 IT MI20070817
(71) Applicant: Farmacetika Limited, The 401 Centre 302 Regent Street London W1B 3HH (GB)
(72) Inventor: Ferrara, Teodosio Antonio, 85027 Rapolla (PZ) (IT)
(74) Representative: Zanoli, Enrico

(57) **Abstract**

The invention concerns the use of 1-camitine and an antagonist of the membrane adenosine receptors, derivatives and/or pharmaceutically acceptable salts thereof in the preparation of drugs with non-toxic effect to inhibit the proliferation of neoplastic cells. Such inhibition is directly proportional to the increase of intracellular ATP.

## Description

The present invention relates to the use of L-carnitine and of an antagonist of the membrane adenosine receptors, or homologs and derivatives thereof, in the preparation of drugs with non-toxic effect to inhibit the proliferation of neoplastic cells. More specifically, the antagonist of the membrane adenosine receptors is suramin.

European patent EP 1 100 589 B1 describes the use of L-carnitine or acetyl-L-camitine in combination or association with taxol in the preparation of an anticancer drug. L-camitine and its acetyl derivative are described as substances that produce a synergic effect on the anticancer activity of taxol and reduce its high toxicity and side effects. These side effects include cumulative toxicity in the peripheral nervous system and neutropenia.

In the literature it is also described how carnitine can reduce the blastogenesis of T lymphocytes caused by phytohemagglutinin (PHA). This effect was studied on human peripheral blood mononuclear cells (PBMCs) in healthy volunteers (Conti et al; Molecular and Cellular Biochemistry; 131: 1-8; 1994). Neoplastic pathology is caused by an altered DNA replication, and therefore cell replication. DNA replication requires energy, which is supplied by ATP. Altered replication, with a nucleotide arrangement that is no longer orderly, is influenced by a shortage of ATP. The aforementioned study by Conti et al showed that L-carnitine - a substance that promotes the increase in ATP - inhibits, in a dose-dependent manner, lymphocytes, isolated from the human peripheral blood of healthy volunteers and stimulated with PHA, through an increase in the concentration of ATP.

US 2005/0014784 A1 describes the use of suramin or analogs thereof or other synthetic substitutes of endostatin to treat certain forms of invasive cancer or metastatic tumors, or more in general pathological conditions for which an angiogenesis-inhibiting treatment is indicated.

However, the need is felt for effective antineoplastic drugs with negligible or reduced side effects. In particular, the need is felt for drugs that are effective in the treatment of leukemias. Leukemias are tumors originating from the bone marrow cells, characterized by the presence of altered leukocytes in the circulating blood. These can be divided into chronic and acute forms.

Chronic Lymphocytic Leukemia (CLL) is caused by uncontrolled proliferation of lymphocytes, which are unable to perform their role in the immune system, so that secondary immunodeficiency also occurs in patients, with increased vulnerability to bacterial, viral and fungal infections. The mainstay of treatment for LLC remains chemotherapy, which is based on the administration of chlorambucil or, more recently, fludarabin.

Acute leukemias are highly malignant and aggressive hematologic neoplasias, characterized by an accumulation of immature cells (blasts) in the bone marrow and peripheral blood. The two principal forms of acute leukemia (myeloid and lymphoblastic) together form 50-60% of all leukemias. Both are very aggressive and characterized by poor prognosis.

The intensification of the chemotherapic doses today seems to have reached the limit of tolerability and this calls for the development of new molecules or new therapeutic approaches. Moreover, an important limitation in the treatment of these neoplasias is represented by intrinsic or acquired drug resistance. The intensification of chemotherapy should be the preferred treatment, but this can cause an unacceptable incidence of important side effects. Moreover, it must be stressed that many cytotoxic drugs are not selective for leukemic cells and therefore also affect normal cells (including hematopoietic cells), causing bone marrow aplasia, the cause of dangerous side effects during the induction phase.

Therefore, at the state of the art the problem of providing a combination or association of drugs that is effective in treating neoplastic pathologies and which have negligible or reduced side effects has still not been solved - or has been solved in an unsatisfactory manner.

A first aspect of the present invention therefore relates to the use of L-carnitine or a derivative thereof of formula (I)

⁺N-(CH₃)-CH₂-CH(OR)- CH₂-COOH X⁻ (I)

Where R = H or a C₂₋₈ alkanoyl group
X = anion of a pharmaceutically acceptable salt
and of an antagonist of the membrane adenosine receptors, or a derivative or salt thereof, pharmaceutically acceptable in the preparation of a drug for treating neoplastic pathologies. According to another aspect of the invention, the antagonist of the membrane adenoside receptors is suramin of formula (II)

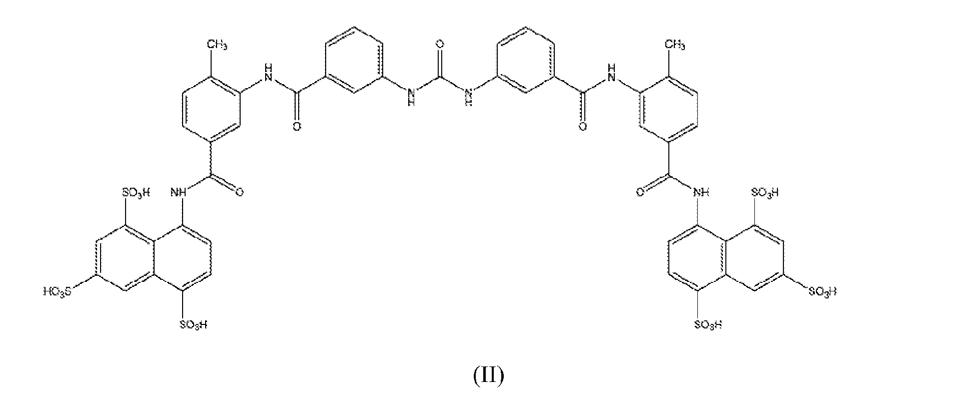

A further aspect of the invention relates to a pharmaceutical composition comprising L-camitine or a derivative thereof of formula (I) and an antagonist of the membrane adenosine receptors or a derivative thereof, or their pharmaceutically acceptable salts.

A further aspect of the invention relates to a pharmaceutical association of L-camitine or a derivative thereof of formula (I) and an antagonist of the membrane adenosine receptors or a derivative or pharmaceutically acceptable salt thereof.

A further aspect of the invention relates to a kit comprising:
- a pharmaceutical composition comprising a therapeutically effective quantity of L-camitine or a derivative thereof of formula (I) or pharmaceutically acceptable salt;
- a pharmaceutical composition comprising a therapeutically effective quantity of an antagonist of membrane adenosine receptors, suramin or a derivative thereof or pharmaceutically acceptable salt.

According to the invention, it has now been found that the combined use, simultaneous or sequential, of L-camitine and of an antagonist of the membrane adenosine receptors, or derivatives thereof and pharmaceutically acceptable salts, is effective in the treatment of certain neoplastic pathologies.

In fact, it has surprisingly been found that the combined use of L-camitine and of an antagonist of membrane adenosine receptors, preferably suramin, determines an increase in the energy metabolism, therefore of ATP, and an increase in cell apoptosis. It has also been found that inhibition of the proliferation of neoplastic cells taken from patients affected by malignant tumors and treated with the composition according to the present invention is due to the increase in intracellular and not in extracellular ATP. This is particularly advantageous as it is known that an increase in extracellular ATP has a toxic effect on the cell. In particular, inhibition of the proliferation of neoplastic cells is directly proportional to the increase in intracellular ATP.

Within the scope of the present invention "pharmaceutical association comprising L-camitine or a derivative thereof of formula (I) and an antagonist of the membrane adenosine receptors or a derivative thereof or their pharmaceutically acceptable salts" is intended both as the composition containing said active ingredients, if necessary prepared extemporaneously, and as the combination of these *in vivo* through simultaneous or sequential administration thereof. The expression "derivatives or pharmaceutically acceptable salts" is intended as any derivative or salt that is both therapeutically effective and does not have toxic effects.

Examples of pharmaceutically acceptable salts of L-camitine are chloride, bromide, iodide, aspartate, acid aspartate, citrate, citrate acid, tartrate, acid tartrate, phosphate, acid phosphate, fumarate, acid fumarate, glycerophosphate, glucosephosphate, lactate, maleate, acid maleate, mucate, orotate, oxalate, acid oxalate, sulfate, acid sulfate, trichloroacetate, trifluoroacetate, methanesulfonate, pamoate, acid pamoate.

Examples of pharmaceutically acceptable salts of suramin are obtained from the following acids: hydrochloric, hydrobromic, sulfuric, nitric, phosphoric, maleic, acetic, salicylic, p-toluenesulfonic, tartaric, citric, formic, malonic, succinic acid.

The pharmaceutical composition or association according to the invention can include other components, such as excipients, pH buffers and preservatives, as it is known in the field.

The pharmaceutical composition or association according to the invention is suitable to be administered in any form, as is known to those skilled in the field.

The invention will now be illustrated with reference to the following examples, relative to ex vivo treatment of cells of patients affected by chronic lymphocytic leukemia.

### EXAMPLES

The protocol for isolation and treatment of cells from patients affected by chronic lymphocytic leukemia comprised the following steps:

### A. Isolation of peripheral blood mononuclear cells (PBMC) from patients affected by chronic lymphocytic leukemia

- Blood sample taken between 8:00 and 9:30 a.m.;
- Blood sample sent to the laboratory to be processed immediately;
- Evaluation of the total volume of the blood received to proceed with 1:2 dilution with sterile saline solution;
- Isolation of the mononuclear cells performed by layering of the blood on a Ficoll-Paque Plus density gradient at a ratio of 1:3, ensuring that the two phases remain separate;
- Centrifugation for 40 minutes at 1800 rpm at room temperature;
- Recovery of the PBMC layers in the interface between the Ficoll and the plasma;
- Washing in saline solution to eliminate residual Ficoll centrifuging at 1200 rpm for 7 minutes at 4°C;
- Washing in RPM1 1640 medium, centrifuging at 1200 rpm for 7 minutes at 4°C;
- Pellet suspension in complete medium, i.e. RPM1 1640 with the addition of L-glutamine 4mM, penicillin at 50U/ml, streptomycin 50 mg/ml and with 10% of FBS (fetal bovine serum);
- Preparation of a 10% cell suspension with Trypan Blue dye and counting of cells under the microscope with Burker camera; cell vitality is also checked using the dye exclusion test;
- Counting of the total number of cells and adjustment of the cell concentration to the required value.

### B. Cell culture

The cells thus isolated were cultured in the complete culture medium, and a culture was prepared placing 1 mL of cell suspension in a 5 mL polypropylene pipette (Falcon) to study cytokine expression and production, and 100 microL in each microplate well. To induce cell blast formation, mitogen phytohemagglutinin (PHA) (20 ug/mL (Pharmacia) selective for T-lymphocytes was added.

The control culture was prepared without adding any mitogenic substance.

The substance to be tested, i.e. L-carnitine and L-carnitine + suramin, at the desired concentration, was added both to cultures with and to those without mitogen. The cultures were incubated in an incubator at 37°C, in a humidified environment with 5% carbon dioxide added.

After 24 hours of incubation the samples were centrifuged; the supernatants were preserved at -20°C, the pellets were resuspended in TRIzol, for subsequent RNA extraction.

All samples obtained were preserved at -80°C until analysis.

### C. Cell proliferation

Evaluation of cell proliferation and therefore of DNA synthesis by isolated and cultured PBMC was performed after stimulation with mitogen and using the Biotrak ELISA system (Amersham Biosciences Europe GmbH, Milan, Italy), based on the measure of incorporation of 5-bromo-2-deoxyuridine (BrdU), pyrimidine analog, into the newly synthesized DNA. 18 hours before the end of incubation 10 microl of culture medium containing BrdU at a final concentration of 10 micromol/L were added to the cultures. At the end of incubation the culture medium was removed by centrifuging the plates at 300 rpm for 10 minutes. Subsequently, the plates were kept in an oven at 60°C for one hour. This was followed by two incubations of 30 minutes each, first in the presence of a fixative required for cell fixation and DNA denaturation, then in the presence of a blocking buffer. After addition of the anti-BrdU antibody, combined with the enzyme peroxidase, the plates were incubated for 90 minutes, after which three washes of 5 minutes each were performed, using PBS. The substrate used for the colorimetric reaction consisted of 3,3',5,5'-tetramethylbenzidine. The reaction was blocked after 15 minutes with H₂SO₄ 1M and the optical density was measured at a wavelength of 450 nanometers, using a plate reader spectrophotometer. The PBMC proliferation values in the different wells were expressed as optical density from which the optical density recorded in the control wells without BrdU was subtracted.

### D. RNA Extraction

After culture the PBMCs were preserved in TRIzol, which permits isolation of total RNA. After defrosting, the samples were resuspended in the TRIzol and left for 5 minutes at room temperature. They were then centrifuged at 1000 rpm for 10 minutes at 6°C and the supernatant was collected in a new pipette. 0.2 mL of chloroform for each mL of initial TRIzol were added to this. It was then vortexed for 15 seconds and left for 3 minutes at room temperature.

Centrifugation was carried pot at 10000 rpm for 15 minutes at 6°C. The aqueous phase (approximately 60% of the total) was removed and transferred to a new pipette.

0.5 mL of isopropyl alcohol per ml of TRIzol were added to the new pipette to precipitate the RNA. It was left at room temperature for 10 minutes and centrifuged at 10000 rpm for 15 minutes at 6°C.

After removing the supernatant, the pellet was washed in 1 mL of 75% cold ethanol to eliminate traces of chloroform. It was energetically stirred and centrifuged at 8000 rpm for 5 minutes at 6°C.

The pellet was left to dry in the air for approximately 5 minutes and then resuspended in 40 microL of sterile DEPC water.

After having dissolved the RNA in water, the concentration was read and the status of the nucleic acid was checked by electrophoretic migration on denatured agarose gel containing 1 % formaldehyde.

The total RNA was preserved in a solution of 0.1 volumes of sodium acetate and 2.5 volumes of absolute ethanol at -20°C.

### E. Reverse Transcriptase PCR Amplification (RT-PCR)

The c-DNA was obtained through a retrotranscriptase reaction by the reverse transcriptase. The reaction took place in the presence of oligonucleotides to synthesize the DNA and of specific primers for the genes to be analyzed. G3PDH was used as ubiquitous reference gene.

The reaction mixture was prepared with the High Capacity cDNA kit by *Applied Biosystem.* The product of the reverse transcriptase was an RNA-DNA hybrid. To eliminate the RNA that was used as a template, 1 µL of RNAsi H, an enzyme of E. coli that destroys the residual RNA molecules, was added. It was placed in the thermal cycler at 37°C for 20 minutes. The cDNA obtained was preserved at -20°C.

### F. Agarose gel electrophoresis

The amplified fragments were separated through electrophoresis on 2% agarose gel containing ethidium bromide. The signal was amplified using the software bio-profile (Vilber Lourmat, Nice, France) and semi-quantitative analysis was performed comparing the signal of the amplified fragments by G3PDH.

### Results of the treatment

These are indicated in the table below, where I.S. indicates the mitogen stimulation index, i.e., the PHA stimulation index.

| Patient | No. of lymphocytes after PHA stimulation | I.S. | % reduction in cell blast formation | |
|---|---|---|---|---|
| | | | Carnitine only | Carnitine+suramin |
| 1 | 35,000,000 | 1.9 | 30 | 49 |
| 2 | 32,000,000 | 3.4 | 65 | 66 |
| 3 | 104,000,000 | 4.4 | 40 | 83 |
| 4 | 52,000,000 | 1.6 | 24.4 | 39.6 |
| 5 | 23,000,000 | 2.3 | 16.5 | 67 |

From the results obtained it can be observed that:
- from the same volume of blood we obtain a different total PBMC concentration in the various patients;
- the response to PHA varies in the different patients;
- carnitine reduces the proliferation induced with PHA in all patients, but in a different manner;
- carnitine in combination or association with suramin enhances this reduction.

There have been described preferred embodiments of the invention, which is susceptible to modifications and variants within the scope of the appended claims.

## Claims

1. Use of L-carnitine or a derivative thereof of formula (I) or pharmaceutically acceptable salt therof
⁺N-(CH₃)-CH₂-CH(OR)- CH₂-COOH X- (I)
Where R = H or a C₂₋₈ alkanoyl group
X = anion of a pharmaceutically acceptable salt
and of an antagonist of the membrane adenosine receptors, or a derivative or pharmaceutically acceptable salt thereof, in the preparation of a drug for treating neoplastic pathologies.

2. Use according to claim 1, **characterized in that** said antagonist of the membrane adenoside receptors is suramin having formula (II), or a derivative or pharmaceutically acceptable salt thereof

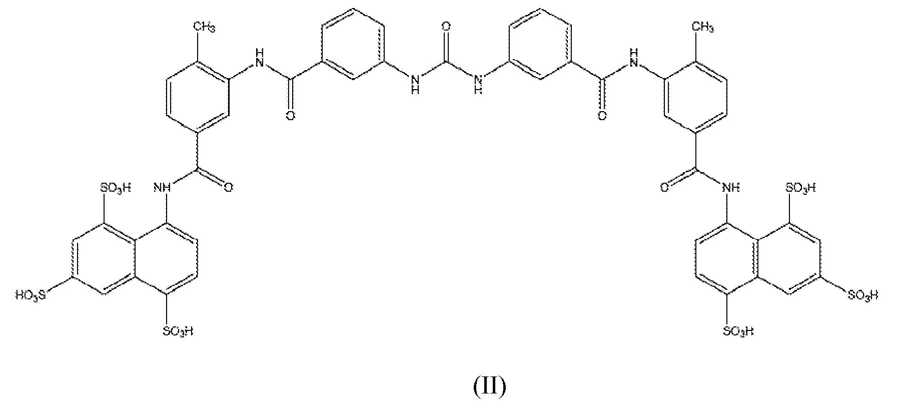

3. Use according to claim 1 or 2, wherein said pathologies involve the presence of neoplastic cells.

4. Use according to claim 3, **characterized by** comprising an inhibition of the proliferation of said neoplastic cells that is directly proportional to the increase in intracellular ATP.

5. Use according to claim 3, wherein said pathology is chronic lymphocytic leukemia.

6. Pharmaceutical composition comprising L-carnitine or a derivative thereof of formula (I) or pharmaceutically acceptable salt thereof
⁺N-(CH₃)-CH₂-CH(OR)- CH₂-COOH X⁻ (I)
Where R = H or a C₂₋₈ alkanoyl group
X = anion of a pharmaceutically acceptable salt
and an antagonist of the membrane adenosine receptors, or a derivative or pharmaceutically acceptable salt thereof.

7. Composition according to claim 6, **characterized in that** said antagonist of the membrane adenosine receptors is suramin of formula (II), or a derivative or a pharmaceutically acceptable salt thereof.

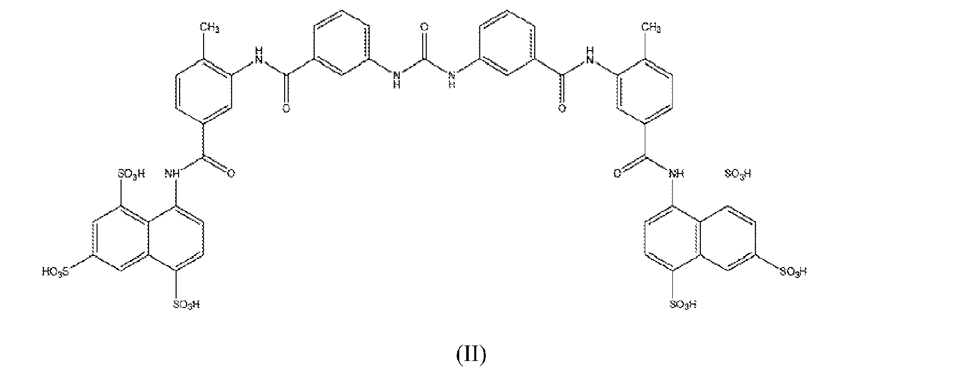

8. Combination of active ingredients for treating neoplastic pathologies comprising L-camitine or a derivative thereof of formula (I) or pharmaceutically acceptable salt thereof
⁺N-(CH₃)-CH₂-CH(OR)- CH₂-COOH X⁻ (I)
Where R = H or a C₂₋₈ alkanoyl group
X = anion of a pharmaceutically acceptable salt
and an antagonist of the membrane adenosine receptors, or a derivative or pharmaceutically acceptable salt thereof.

9. Combination according to claim 8, **characterized in that** said antagonist of the membrane adenosine receptors is suramin of formula (II), or a derivative or pharmaceutically acceptable salt thereof

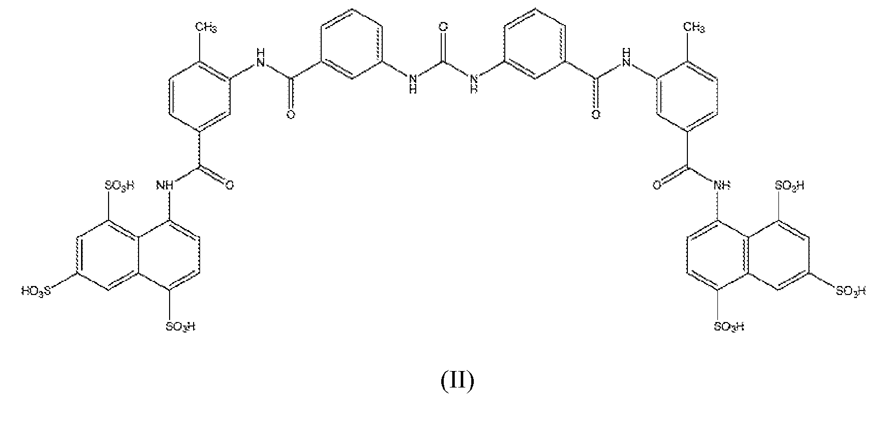

10. Use of the combination according to claim 9 in the preparation of a drug for treating neoplastic pathologies.

11. Use according to claim 10, wherein said pathology involves the presence of neoplastic cells.

12. Use according to claim 11, **characterized by** comprising an inhibition of the proliferation of said neoplastic cells that is directly proportional to the increase in intracellular ATP.

13. Use according to claim 11, wherein said pathology is chronic lymphocytic leukemia.

14. Kit comprising:
a pharmaceutical composition comprising a therapeutically effective quantity of L-camitine or a derivative thereof of formula (I) or pharmaceutically acceptable salt thereof
⁺N-(CH₃)-CH₂-CH(OR)- CH₂-COOH X⁻ (I)
Where R = H or a C₂₋₈ alkanoyl group
X = anion of a pharmaceutically acceptable salt
and a pharmaceutical composition comprising a therapeutically effective quantity of an antagonist of the membrane adenosine receptors, or a derivative or a pharmaceutically acceptable salt thereof.

15. Kit according to claim 14, **characterized in that** said antagonist of the membrane adenosine receptors is suramin of formula (II), or a derivative or a pharmaceutically acceptable salt thereof:
